(19) Europäisches Patentamt
European Patent Office
Office européen des brevets

(11) Publication number: **0 015 631**
**B1**

(12) # EUROPEAN PATENT SPECIFICATION

(45) Date of publication of patent specification: **07.12.83**

(51) Int. Cl.³: **C 07 D 249/12**

(21) Application number: **80200247.7**

(22) Date of filing: **29.06.78**

(54) **((4,5-Dihydro-5-thioxo-1H-1,2,4-triazol-3-yl)thio)acetic acid and its salts.**

(30) Priority: **30.06.77 US 811642**

(43) Date of publication of application:
**17.09.80 Bulletin 80/19**

(45) Publication of the grant of the patent:
**07.12.83 Bulletin 83/49**

(84) Designated Contracting States:
**BE CH DE FR GB LU NL SE**

(56) References cited:
**DE - A - 2 016 082**
**DE - A - 2 016 083**

(73) Proprietor: **SMITHKLINE BECKMAN CORPORATION**
**1 Franklin Plaza**
**Philadelphia Pennsylvania 19101 (US)**

(72) Inventor: **Berges, David Alan**
**18 Buckwalter Road**
**Phoenixville, Pennsylvania 19460 (US)**

(74) Representative: **Waters, David Martin, Dr.**
**P.O. Box 39**
**Welwyn Garden City, Hertfordshire AL7 1EY (GB)**

**EP 0 015 631 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# [(4,5-Dihydro-5-thioxo-1H-1,2,4-triazol-3-yl)thio] acetic acid and its salts

This invention relates to [(4,5-dihydro-5-thioxo-1*H*-1,2,4-triazol-3-yl)thio] acetic acid, its alkali metal and ammonium salts.

Our European Patent Application No. 783001035 discloses and claims certain cephalosporin derivatives. The compounds of this invention are useful as intermediates in the preparation of these cephalosporins.

Accordingly the present invention provides [(4,5-dihydro-5-thioxo-1*H*-1,2,4-triazol-3-yl)thio] acetic acid, its alkali metal and ammonium salts.

The acetic acid derivative of this invention can exist in several tautomeric forms one of which is shown in formula (III):—

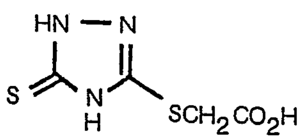

(III)

However it is to be understood that all the tautomeric forms of this compound are within the scope of the invention.

[(4,5 - Dihydro - 5 - thioxo - 1*H* - 1,2,4 - triazol - 3 - yl)thio] acetic acid can be used in the preparation of cephalosporin derivatives as disclosed in our European Patent Application No. 783001035, Publication No. 0000285.

The following Example illustrates the invention.

## EXAMPLE

To a suspension of 5.48 g. (40 mmol) of 1,2,4-triazolidine-3,5-dithione, monohydrazine salt in 80 ml. of tetrahydrofuran and 80 ml. of dimethylformamide was added a solution of 6.7g (40 mmol) of ethyl bromoacetate in 20 ml. of tetrahydrofuran. The mixture was stirred at room temperature for 1—1/2 hours, and then at 50° C. for 1/2 hour. The solution was filtered and the filtrate was concentrated to 80 ml., diluted with 250 ml. of water and extracted with diethyl ether. The extract was dried ($MgSO_4$) and evaporated to dryness to give a residue which was crystallized from methanol and water to give 5.76 g. (65% yield) of ethyl [(4,5-dihydro-5-thioxo-1*H*-1,2,4-triazol-3-yl)thio]acetate. mp 134—6° C.

A solution of 2.19 g. (10 mmol) of ethyl [4,5-dihydro - 5 - thioxo - 1*H* - 1,2,4 - triazol -3 - yl)thio]acetate in 50 ml. of 5% sodium hydroxide was heated at reflux for 1 hour. After thorough cooling, the mixture was filtered, and the filtrate was washed with ethyl acetate. The aqueous layer was separated, acidified to pH 1 and extracted with ethyl acetate. The extract was evaporated *in vacuo* to dryness. The residue was crystallized from chloroform to give 1.43 g. (75% yield) of [(4,5-dihydro-5-thioxo-1*H*-1,2,4-triazol-3-yl-)thio]acetic acid.

**Claim**

[(4,5 - Dihydro - 5 - thioxo - 1*H* - 1,2,4 - triazol - 3 - yl)thio]acetic acid, its alkali metal and ammonium salts.

**Patentanspruch**

[(4,5-Dihydro-5-thioxo-1*H*-1,2,4-triazol-3-yl)-thio]-essigsäure, ihre Alkalimetall- und Ammoniumsalze.

**Revendication**

L'acide [(4,5-dihydro-5-thioxo-1*H*-1,2,4-triazol-3-yl)thio]acétique, ses sels de métaux alcalins et d'ammonium.